(19) Europäisches Patentamt
European Patent Office
Office européen des brevets

(11) **EP 4 036 930 A1**

(12) **EUROPEAN PATENT APPLICATION**

(43) Date of publication:
**03.08.2022 Bulletin 2022/31**

(21) Application number: **21168593.8**

(22) Date of filing: **15.04.2021**

(51) International Patent Classification (IPC):
*G16H 50/00* (2018.01)     *G06T 19/00* (2011.01)
*A61B 5/367* (2021.01)     *A61B 5/06* (2006.01)
*A61B 5/0538* (2021.01)     *A61B 5/00* (2006.01)

(52) Cooperative Patent Classification (CPC):
**A61B 5/367; A61B 5/0538; A61B 5/061;
A61B 5/6852; G16H 20/40; G16H 40/63;
G16H 50/50**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.01.2021 US 202163143590 P**

(71) Applicant: **Koninklijke Philips N.V.
5656 AG Eindhoven (NL)**

(72) Inventors:
• **IBRAGIMOV, Zalman
  30889 Caesaria (IL)**
• **GLUHOVSKY, Leonid
  30889 Caesaria (IL)**
• **MAGADLA, Muhamad
  30889 Caesaria (IL)**

(74) Representative: **Philips Intellectual Property &
Standards
High Tech Campus 52
5656 AG Eindhoven (NL)**

(54) **IMAGE RECONSTRUCTION METHOD FOR DIELECTRIC ANATOMICAL MAPPING**

(57)     A mechanism for generating a position space anatomical model of an anatomical cavity. Electrical responses, of an electrode positioned within the anatomical cavity, are obtained. A response space anatomical model is constructed based on the electrical responses. The response space anatomical model is then converted into a position space anatomical model using a mapping function.

FIG. 5

EP 4 036 930 A1

**Description**

FIELD OF THE INVENTION

The present invention relates to the field of dielectric imaging also referred to as electroanatomical mapping of an anatomical region of a subject using electric fields applied externally to the subject and sensed by one or more electrodes located within the subj ect.

BACKGROUND OF THE INVENTION

Dielectric imaging or electroanatomical mapping is advantageously used in for example medical imaging and/or treatment of e.g. cardiac illness such as atrial fibrillation using ablation procedures as known in the art.
With dielectric imaging or electroanatomical mapping, two or more crossing electrical fields are induced inside of the body of a subject by an array of electrodes positioned on the outside of the body of a subject ("external electrodes"), such as on the surface of the body of the subject. These electric fields induce position dependent electrical responses (the electrical response data), such as a voltage response, in electrodes placed within the body (an "internal electrode") of the subject. The position of an internal electrode can thereby be tracked by monitoring the electrical response of the electrode to these electric fields while moving the electrode. In particular, an appropriate mapping function can be used to map or transform the set of electrical responses so recorded to a set of positions within position space, being a multidimensional (Euclidian/Cartesian) co-ordinate system representing true spatial positioning. From the recorded positions data an anatomical model of the internal anatomy of the subject may be (re)constructed for example in the form of a mesh representing the surface of an internal cavity of the body. Construction of the anatomical model is possible because it can be assumed that the internal electrodes will only be located within cavities of the subject, thereby allowing the bounds of the cavity to be constructed.
Examples of mechanisms for constructing a model of the internal anatomy of the subject based on the response of internal electrodes to crossing electric fields induced by external electrodes is disclosed by US patent 5697377 or EP patent application 3,568,068.
There is an ongoing desire to improve the mapping and/or reconstruction of the anatomical model in terms of accuracy and/or speed.

SUMMARY OF THE INVENTION

The inventors have recognized that mapping of electrical response data to the position data may be computationally heavy and time consuming for example due to non-linearity effects in the electrical response data. This makes real-time reconstruction of the anatomical model difficult especially when resolution of the model is relatively high and/or there is a large amount of electrical response data available.
The current disclosure aims to, inter alia, reduce or alleviate one or more of the aforementioned problems.
According to examples in accordance with an aspect of the invention, there is provided a computer-implemented method as defined in claim 1.
As is common in the art, a mapping function is defined based on a relatively large number of electrical responses in an electrical response space (V-space) and is subsequently operated on the V space data to generate an anatomical model in a position space (R space). Then an anatomical model in the form of a mesh is created in R space. This process may be computationally heavy and concomitantly take an increased amount of time to be conveniently used in real time mapping procedures.
The amount of time for mapping may be reduced by first generating a representation of the anatomical model in V space (referred to as the response space anatomical model or "preanatomical model"), preferably using a scaling action such that the representation is based on characteristics of the V space data and of the R space data. Thus, the three-dimensional R space characteristics can be effectively translated into a mesh in the V space forming the response space anatomical model. Operating the mapping function may now only be done on the response space anatomical model to generate the final, position space anatomical model in R space. The final operation of the mapping function can thus be done on a smaller set of data, i.e. the mesh data (as the number of vertices of the mesh will likely be less than the number of electrical responses) instead of the V space data, Use of a scaling action provides a further advantage in meaning that this process can be performed with reduced loss of R space characteristics defined by the three-dimensional R space data.
The present disclosure proposes a computer-implemented method for generating a position space anatomical model, being an anatomical model in a multidimensional position space, of an anatomical cavity of a subject in which an interventional device comprising one or more electrodes is positioned.
The computer implemented method comprises: receiving or obtaining electrical responses, defining a response space, of one or more electrodes of the interventional device to crossing electric fields induced within the cavity; generating a response space anatomical model using the obtained electrical responses; and generating the position space anatomical model by converting the response space anatomical model into the multi-dimensional position space using a first mapping function.

The computer-implemented method may further comprise providing a representation of at least part of the position space anatomical model to a user interface.

In some examples, the step of generating the response space anatomical model comprises: determining linear scaling factors that scale each electrical response to an approximate position, for the electrode associated with that electrical response, in the multidimensional position space; determining scaled electrical responses from the electrical responses using the linear scaling factors; generating a scaled response space anatomical model in the electrical response space from the scaled electrical responses; and generating the response space anatomical model by descaling the scaled response space anatomical model using the linear scaling factors.

Generating a scaled response space anatomical model allows information about position space characteristics to be taken into account when generating the anatomical model. This is because the scaling effectively acts as a way to introduce positon space information (through the linear scaling) into the response space. A more accurate anatomical model is thereby generated.

In some examples, the step of determining linear scaling factors comprises: determining positions of the plurality of electrodes within the multi-dimensional position space from the electrical responses using a second mapping function; and determining linear scaling factors between the electrical responses, in the response space, and the corresponding positions in the multi-dimensional position space.

The step of determining linear scaling factors may comprise dividing the positions, in the multi-dimensional position space, by their corresponding electrical responses. This approach facilitates a mechanism for estimating or establishing a linear (e.g. scalar) correspondence between a position in the position space and a response in the response space. The quotients of the divisions may be averaged in order to generate average scaling factors, which can then act as the linear scaling factors.

In other examples, the step of determining linear scaling factors comprises determining a linear mapping function that maps a response, in response space, to a position in position space using the linear scaling factors. Thus, linear scaling factors can be directly obtained in the process of obtaining a linear mapping function. Approaches for generating a linear mapping function are well established in the art, and generate scaling factors to be applied to a response to map it to a relative position in the position space (whilst preserving known characteristics of the responses as best as possible, e.g. to minimize an average error).

The first mapping function may be a mapping function obtained by relating electrical responses of pairs of electrodes to positions in the position space using one or more inter-electrode distances between the pairs of electrodes as constraints. In particular, the first mapping function may be non-linear.

The response space anatomical model and the position space anatomical model both comprise of a mesh having triangular or quadrilateral cell shapes. Use of such meshes facilitates ease of mapping from the response space to the position space, as the vertices of the response space mesh represent points to be mapped from the response space to the position space.

The electrical responses may comprise electrical responses within a predetermined part of an anatomical cycle of the subject. This approach can help account for anatomical cycles (e.g. respiratory and/or circulatory cycles) of the patient, which may affect the electrical responses, which effect could lead to a more inaccurate anatomical map being generated (as the mapping function may be unable to take the change of an electrical response due to anatomical cycle activity into account).

The computer-implemented method may comprise: controlling generation of electrical signals and application of these signals to a set of external body electrodes which when applied to the surface of the subject cause the crossing electric fields to be induced within the cavity.

The computer-implemented method may comprise controlling the obtaining of the electrical responses of the one or more electrodes.

There is also proposed a computer program product comprising computer program code means which, when executed on a computing device having a processing system, cause the processing system to perform all of the steps of any herein claimed method.

Similarly, there is also proposed a computer-readable (storage) medium comprising instructions which, when executed by a computer or processing system, cause the computer or processing system to carry out (the steps of) any herein described method. There is also proposed computer-readable data carrier having stored thereon the computer program (product) previously described. There is also proposed a data carrier signal carrying the computer program (product) previously described.

There is also proposed an anatomical model generator for generating an anatomical model in a multidimensional position space of an anatomical cavity of a subject in which an interventional device comprising one or more electrodes is positioned.

The anatomical model generator comprises: an input interface configured to receive or obtain electrical responses, defining a response space, of one or more electrodes of the interventional device to crossing electric fields induced within the cavity.

The anatomical model generator also comprises a data processor configured to: generate a response space anatomical model using the obtained electrical responses; and generate the position space anatomical model by converting the response space anatomical model into the multi-dimensional position space using a first mapping function,

The anatomical model generator may further comprise an output interface configured to provide a representation of at least part of the position space anatomical model to a user interface.

In some examples, the data processor is configured to generate the response space anatomical model by: determining linear scaling factors that scale each electrical response to an approximate position, for the electrode associated with that electrical response, in the multidimensional position space; determining scaled electrical responses from the electrical responses using the linear scaling factors; generating a scaled response space anatomical model in the electrical response space from the scaled electrical responses; and generating the response space anatomical model by descaling the scaled response space anatomical model using the linear scaling factors.

The data processor may be configured to determine linear scaling factors by: determining positions of the plurality of electrodes within the multi-dimensional position space from the electrical responses using a second mapping function; and determining linear scaling factors between the electrical responses, in the response space, and the corresponding positions in the multi-dimensional position space.

There is also proposed an anatomical model system comprising: the anatomical model generator previously described, wherein the anatomical model generator comprises the output interface; and the user interface configured to receive the representation of at least part of the position space anatomical model and display the received representation. The anatomical model system may be alternatively labelled a processing system.

The user interface may receive the anatomical model or a representation thereof from the output interface of the anatomical model generator.

There is also proposed a processing system comprising the anatomical model generator or the anatomical model system previously described.

The processing system further may further comprise an electric field generator, configured to control the electric fields induced within the anatomical cavity. The electric field generator may communicate with one or more electrodes (positioned on a surface of the subject) in order to generate the electric fields. The processing may itself comprise the electrodes that generate the electric fields.

In some examples, the processing system comprises an electrode response sensing device, configured to sense or sample one or more electrical responses of the electrodes of the interventional device to the electric fields. The electrode response sensing device may be communicatively coupled to the electrodes of the interventional device (e.g. by one or more wires) and configured to sample the one or more electrical responses. In particular, the electrode response sensing device may be configured to sense or sample, at each of a plurality of time points, a set of measurements from each electrode of the interventional device (the set of measurements forming

BRIEF DESCRIPTION OF THE DRAWINGS

For a better understanding of the invention, and to show more clearly how it may be carried into effect, reference will now be made, by way of example only, to the accompanying drawings, in which:

    Figure 1 illustrates positions for a set of external electrodes positioned on a subj ect;
    Figure 2 illustrates a co-ordinate system for defining a direction of electric fields generated by the external electrodes;
    Figure 3 illustrates a dielectric imaging system;
    Figure 4 illustrates an approach for reconstructing an anatomical model from a point cloud;
    Figure 5 illustrates a method according to an embodiment; and
    Figure 6 illustrates an anatomical model generator.

DETAILED DESCRIPTION OF THE EMBODIMENTS

The invention will be described with reference to the figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the figures are merely schematic and are not drawn to scale. It should also be understood that the same reference numerals are used throughout the figures to indicate the same or similar parts.

The invention provides a mechanism for generating a position space anatomical model of an anatomical cavity. Electrical responses, of an electrode positioned within the anatomical cavity, are obtained. A response space anatomical model is constructed based on the electrical responses. The response space anatomical model is then converted into a position space anatomical model using a mapping function.

The present disclosure is based on a realization that a position space anatomical model can be generated by mapping a response space anatomical model to the position space, rather than mapping all responses to the position space before directly generating a position space anatomical model. This approach can reduce the number of mappings that need to be performed, as a model is typically formed of fewer individual points in space (e.g. vertices of a mesh) than are required to generate the model.

Embodiments of the invention may be employed in any suitable scenario in which an anatomical model of an anatomical cavity is generated based on the response(s) of one or more internal electrodes (mounted on an interventional device) to electric fields induced within the anatomical cavity. One example scenario could be the mapping of a heart of chambers thereof and surrounding blood vessels using an interventional device, such as a catheter, inserted into the cardiovascular system.

For the purposes of contextual understanding, the principle and purpose of generating an anatomical model of an anatomical cavity, according to standard approaches, involving the use of electric fields within a subject, will be hereafter described.

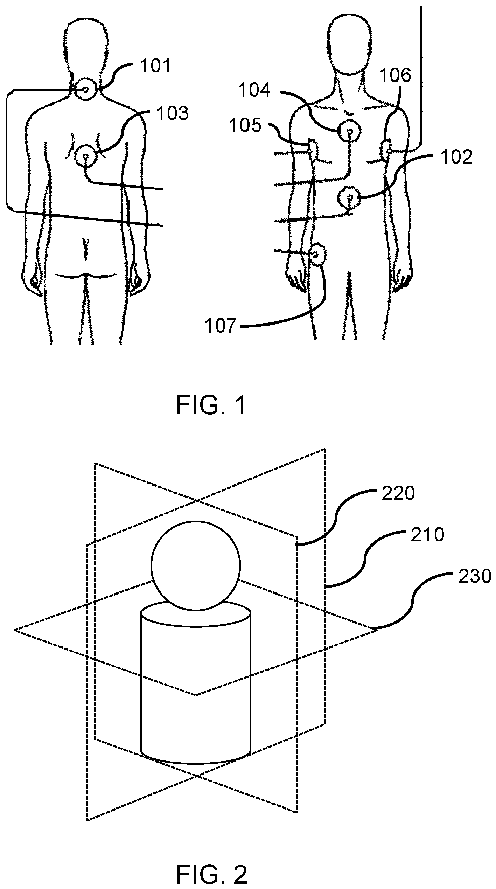

Figure 1 illustrates positions for a set of external electrodes positioned on a subject 190. External electrodes are electrodes that are positioned externally to a subject, and are contrasted with internal electrodes that are positionable within the subject, for example, as part of an interventional device such as a catheter.

The illustrated set of external electrodes comprises a first external electrode 101, a second external electrode 102, a third external electrode 103, a fourth external electrode 104, a fifth external electrode 105 and a sixth external electrode 106. These electrodes are preferably located on the subject such that they can provide crossing electric fields within the subject as described hereinafter. The illustrated set further comprises a reference electrode 107, which can act as a "ground" for defining a base/background level of electric field activity in the subject.

An electric signal provided to each external electrode is controlled to thereby define crossing electric fields between the electrodes. In particular, the first 101 and second 102 external electrodes form a first pair of external electrodes ("external electrode pair"), and signals provided to the first external electrode pair can be controlled to induce a first electric field therebetween. The third 103 and fourth 104 external electrodes form a second pair of external electrodes, and signals provided to the second external electrode pair can be controlled to induce a second electric field therebetween. The fifth 105 and sixth 106 external electrodes form a third pair of external electrodes, and signals provided to the third external electrode pair can be controlled to induce a third electric field therebetween. The pairs of electrodes are preferably arranged such that they provide substantially mutually orthogonal crossing electrical fields.

Electric signals provided to each external electrode, and in particular to each pair of electrodes, can control the frequency and/or magnitude/strength of the crossing electric fields. The electric signals supplied to the electrodes may be controlled by an electric field generator (not illustrated in Figure 1).

The crossing electric fields are usable to track or identify a location of electrodes positioned within the crossing electric fields. In particular, an electrical response of an electrode to each electric field will change as a position within the electric field changes (e.g. a distance from a source/sink of the electric field changes). The electrical response of an electrode to the crossing electric fields can be mapped or associated with a particular position within the electric fields.

In other words, an electrical response (which can be alternatively labelled a "measurement") of the internal electrode to the crossing electric fields can be processed to determine a predicted position of the internal electrode (e.g. and therefore of any interventional device comprising the internal electrode) within an anatomical cavity of the subject.

In particular, each electric field may be controlled to have a particular/different frequency. This facilitates determination of the predicted position of an electrode with respect to each electric field, by assessing the electrical response of the electrode to the particular frequency of the electric field. This information can be used to effectively identify or predict the position of the electrode with respect to a co-ordinate system defined by the electric fields.

One of the external electrode pairs may be omitted, e.g. if only two crossing electric fields are desired (e.g. for performing a 2-dimensional tracking process).

Figure 2 schematically illustrates a co-ordinate system for defining a direction of electric fields generated by the external electrodes. The co-ordinate system defines three cardinal planes 210, 220, 230. The crossing electric fields may, for instance, be optimally positioned when the direction of each electric field is parallel to a respective cardinal place 210, 220, 230.

The intent of the crossing electric fields, e.g. such as those generated using the set illustrated by Figure 1, is to facilitate identification of the position of an electrode (or group of electrodes) with respect to a co-ordinate system, such as that illustrated by Figure 2.

A more complete example of how to track the position of an internal electrode with respect to crossing electric fields how to further exploit this information for the construction of an anatomical model of an anatomical cavity of the subject, is hereafter described for the purposes of improved contextual understanding.

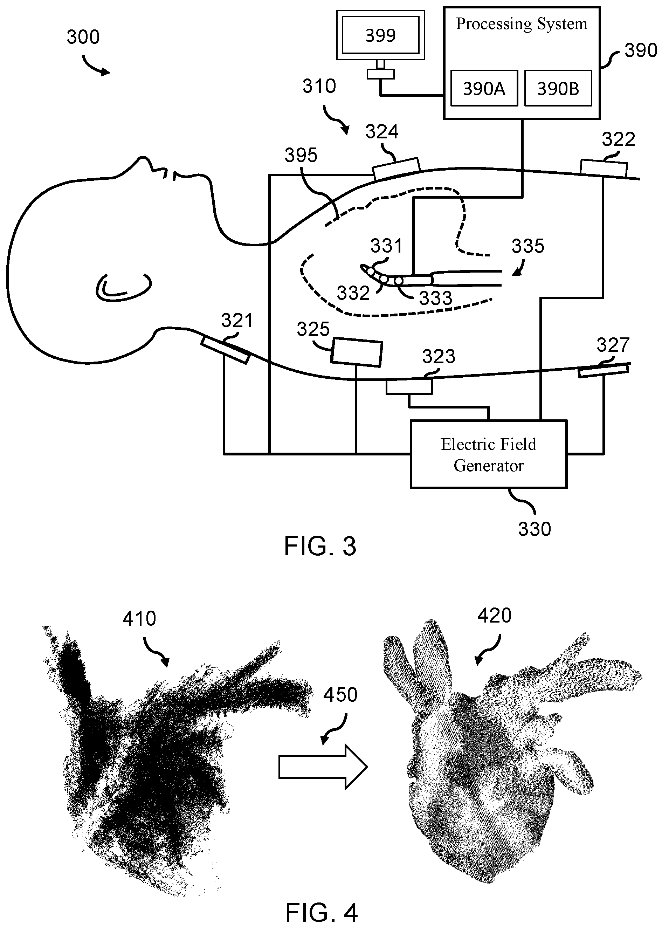

Figure 3 conceptually illustrates a processing arrangement 300 for generating a mapping function for predicting the position of an electrode within an anatomical cavity and for generating an anatomical model of an anatomical cavity within a subject. The concept of the present disclosure is primarily directed towards the process of generating the anatomical model.

In particular, the processing arrangement 300 may be configured to generate an anatomical model (of an anatomical cavity, such as a blood vessel and/or chamber) using a dielectric imaging process.

The dielectric imaging system 300 comprises an exemplary electric field generating arrangement 310 and a processing system 390. The processing system 390 may contain a mapping function generator 390A (i.e. a "processor") and/or an anatomical model generator 390B according to an embodiment, although these elements may be alternatively positioned externally to the processing system 390.

Although not separately shown, the processing system 390 may also comprise a further electric field generator and measurer that is controlled by the processing system to generate and provide electrical signals to the electrodes of an interventional device (when connected to the processing system) and to detect/measure electrical signals of these electrodes.

Thus, in some examples, the processing system 390 may perform at least some of the functions of an electric field generator 330, described in more detail below.

The processing system may also comprise an electric signal measurer (not shown) configured to measure and/or sample the electrical response(s) of the electrode(s) of the interventional device to crossing electric fields, as explained in more detail below.

The electric field generating arrangement 310 comprises a set of external electrodes 321, 322, 323, 324, 325, 327 for being positioned externally with respect to the subject 390 (e.g. as electrode patches provided on a skin of the subject). The set 310 of external electrodes may comprise a plurality of electrode pairs angled with respect to one another (e.g. positioned orthogonally to one another), so that any electric fields generated by the electrode pairs are angled with respect to one another. These electrode pairs may comprise a first electrode pair (formed of a first 321 and second 322 external electrode), a second electrode pair (formed of a third 323 and fourth 324 external electrode) and a third electrode pair (formed of a fifth 325 and sixth (not visible) external electrode). One of more of these electrode pairs may be omitted. The set of external electrodes may also comprise a reference electrode 327. Thus, the external electrodes are placable in a similar manner to the set of external electrodes illustrated in Figure 1.

The electric field generating arrangement 310 also comprises an electric field generator 330, which is adapted to generate and/or control (characteristics of) an electric signal (e.g. voltage and/or current) supplied to each external electrode. The electric field generator may, in some example, form part of the processing system 390. The electric field generating arrangement 310 is configured to generate a plurality of (here: three) crossing (intra-body) electrical fields using the external electrodes. This is performed using appropriate control of the electric signals provided to each external electrode, for example, by providing alternating constant currents such as constant amplitude currents to each of the electrode pairs.

In particular, each electrode pair may be appropriately controlled to induce an electric field between each electric pair. Thus, where there are three electrode pairs, three electric fields may be generated. Preferably, the frequency of each generated electric field is controlled to be different, to facilitate increased ease in identifying a relative location of an electrode positioning within the crossing electric fields.

The generated electric fields can be used to define or establish a relative location of an (internal) electrode positioned within the crossing electric fields. In particular, as previously explained, the (electrical) response of an internal electrode to the electric fields changes as the predicted position of the internal electrode moves about the subject. This is at least partly because the induced electrical fields' distribution is inherently inhomogeneous due to the different dielectric properties and absorption rates (related to conductivity) of the interrogated tissues of the subject under investigation. The principle of crossing electric fields thereby facilitates tracking of the predicted position of the internal electrode using an anatomical model generator 390 that monitors an electrical response of any internal electrodes to the crossing electric fields, e.g. by mapping the (electrical) response of an electrode to a predicted position within the subject or using a suitable mapping/transfer function, e.g. a "V2R function" described below to determine the predicted position(s) of the electrodes.

By way of further explanation, for the purposes of improved conceptual understanding, if the crossing electric fields are controlled to have a different frequency with respect to one another, then the electrical response of the internal electrode to each frequency could be used to determine a relative distance between each source/sink of the corresponding electric field. This principle can be used to effectively triangulate the predicted position of the internal electrode within the crossing electric fields.

For instance, if there are three external electrode pairs, positioned to emit electric fields ($E_1$, $E_2$, $E_3$) of different

frequencies that are angled (e.g. near-orthogonal) with respect to one another, a voltage response ($V_1$, $V_2$, $V_3$ or $v_z$, $v_y$, $v_z$) of an internal electrode identifying a voltage (e.g. between the electrode and the reference electrode or between the electrode and the electrode generating the electric field) at each of these three frequencies, will differ depending upon position within the anatomical cavity.

Thus, an electrical response of an electrode may comprise two or more values, e.g. exactly three values, (e.g. voltage measurements), each value representing a measurement of an electrical parameter responsive to changes of a different electric field induced within the anatomical cavity. Here, each value represents a voltage measurement at a particular frequency (being the frequency of the corresponding electric field). Other suitable electrical measurements could be used instead.

The skilled person will appreciate that determining the position of internal electrodes 331, 332, 333 also facilitates determining the position, orientation and/or angle of an interventional device 325 that mounts the electrodes. In particular, a positional relationship of the electrodes on the interventional device may be known/predetermined, and used to derive an orientation of the interventional device (e.g. define an axis along which the interventional device lies).

Other forms of electrical response for an internal electrode (e.g. an impedance response or a capacitive response, e.g. indicating change in impedance/capacitance between the internal electrode and an external electrode) will be apparent to the skilled person. In such examples, each value of the response may represent an impedance/capacitance measurement at a particular frequency or with respect to a particular electrode emitting a particular electric field. Some electrical responses may include different electric measurements, e.g. a response may contain a voltage measurement, an impedance measurement and/or a capacitance measurement.

The electric field generator 330 may be configured to control the electric fields, generated using the set of external electrodes, to operate in the frequency range of 10kHz -100kHz, e.g. between 10kHz and 25kHz. These frequency ranges is particularly useful for ensuring penetration of a subject, whilst providing a frequency range that attenuates in human tissue without significant damage/injury to the tissue. A lower frequency range (e.g. between 10kHz and 25kHz, e.g. between 10kHz and 20kHz) also benefits from a reduced impact of noise. This facilitates the measurement of more localized and precise electrical responses, with reduced interruption from external sources.

The reference electrode 327 serves as an electric reference for all measurements taken by electrodes, e.g. as a reference for the electrical response of the internal electrodes.

The electrical response of two or more internal electrodes mounted upon a single interventional device can be used to construct (and update) the mapping function used to map an electrical response of the internal electrode (i.e. in a response space) to a predicted position within the anatomical cavity (e.g. within some (Euclidian/Cartesian) position/physical space).

An exemplary process for generating or constructing a mapping function using a mapping function generator 390A is hereafter described.

As previously mentioned, the mapping function generator makes use of a plurality of internal electrodes 331, 332, 333 to be positioned within the anatomical cavity (e.g. electrodes positioned on an interventional device 335 to be inserted into the anatomical cavity). A spatial relationship (e.g. a distance) between each of the internal electrodes may be predetermined and/or known.

The mapping function generator 390A is configured to receive (and optionally provide) signals (e.g. at an input interface) from/to the internal electrodes 331, 332, 333 to determine an electrical response of the internal electrodes to the crossing electric fields.

The electrical response of the internal electrode(s) (e.g. to externally applied electric fields by the external electrodes) is/are then iteratively recorded or sampled for different positions and/or orientations of the interventional device with the body cavity explored, i.e. to obtain or sample one or more "measurements" from each internal electrode. For instance, each sampled response may contain three measurements each corresponding to a respective electric field. This sampling may be performed by an electrode response sampling device (not shown), which may form part of the processing system 390.

It will be understood that this process generates a temporal sequence of sets of one or more electrical responses, each set of one or more electrical responses being obtained at a same point in time (i.e. when the interventional device is at a same position).

Conceptually, each electrical response may itself represent a "point" (e.g. a location defined by a single set of co-ordinates) within a particular Euclidian space system, i.e. a "response space". Where the electrical response is a voltage response, this may be known as the "V-space". The electrical response may, for instance, comprise two or more values responsive to respective electric fields (e.g. filtered by frequency of the electric fields). Each value may represent a respective co-ordinate value in the response space. For instance, if each value represents a voltage measurement (at a respective frequency of a specific electric field), then each electrical response can be represented by a point in a voltage space (V-space).

The mapping function generator can then repeatedly define/update and apply a mapping function or transfer function (e.g. a "V2R function") that transforms each recorded electrical response to Euclidian/Cartesian coordinates in a "real", physical space or position space (R-space), whilst ensuring known properties and/or spatial relationships of the internal electrodes and/or interventional device 335 (e.g. electrode spacing and electrical weight length) and, optionally, a set of other constraints are maintained.

A position space, physical space or R-space represents a "true" position space or physical space, i.e. where a distance between two different points of an axis of the R-space directly represents a physical distance. This differs from the response space, in which a distance between two different points of an axis does not directly represent a physical distance (but rather, represents a change in an electric parameter).

In some examples, the mapping/transfer function can effectively learn and linearize the distorted relation between the measured responses and the actual position in three dimensions by taking the known inter-electrode distances as a reference. In particular, responses of different electrodes captured at a same time must (in the position space) be spaced apart by the known inter-electrode distances. Moreover, if an internal electrode has an electrical response matching a previously measured electrical response, then the internal electrode can be assumed to be in a same position. In this way, the electrical responses can be determined and used to improve the mapping/transfer function.

Put another way, the mapping function is configured to relate electrical responses of pairs of electrodes to positions in the position space using one or more inter-electrode distances between the pairs of electrodes as constraints. Preferably, this mapping function is non-linear to account for the non-linearity of the electric fields within the anatomical cavity (caused by differences in the dielectric constant of different tissues or absence of tissue).

In other words, the mapping/transfer function effectively determines or predicts a relative/predicted position/location of an electrode (when the corresponding response was obtained) in a Euclidian/Cartesian and/or multidimensional space or co-ordinate system ("R-space") representing a position space. Thus, the mapping function transfers from a response space to a position space. In this way, an R-space cloud of points (known Euclidian/Cartesian co-ordinates) can be built up and updated as the internal electrodes are moved within the anatomical cavity. This R-space cloud of points is then iteratively analyzed in order to iteratively update the mapping function to adhere to the known properties of the internal electrodes and/or interventional device, such as a spatial relationship of the internal electrodes.

A more detailed description of examples of the aforementioned process may be found in WO2018130974.

Other and/or more complete descriptions and/or embodiments of the process for generating the mapping function are disclosed by the European Patent Applications EP 0775466 A2, EP 3568068 A1 and EP 3607879 A1. Other example mapping function may, for instance, make use of machine-learning process and/or algorithms (e.g. be machine-learning processes themselves).

As the electric field strength of the electric fields is non-linear, it is preferred that the mapping function used is also non-linear, to improve the accuracy of mapping from the response space to the position space.

The predicted positions of an internal electrode or internal electrodes may also be used to construct an anatomical model of an anatomical cavity (i.e. a cavity in which the interventional device is able to move). This process is called a reconstruction process (or a dielectric imaging process), and may be performed by the anatomical model generator 390B.

Broadly, the anatomical model generator 390B may build an anatomical model of an anatomical cavity 395 (e.g. a chamber, vessel or void) within a subject by processing mapped positions/points in the position space.

In particular, using an cloud of points in the position space, such as the cloud of points in the position space produced during the construction of the mapping function or a cloud of points generated by processing (with the mapping function) responses obtained using a different interventioanl device (i.e. not necessary the same device used to generate the mapping function), a reconstruction algorithm generates an anatomical model of the (investigated part of the) anatomical cavity. The anatomical model may, for example, be a 3D surface that depicts or models the (known bounds of) the anatomical cavity.

In particular, generating an anatomical model may comprise processing a position space (R-space) cloud of points to define the position of the bounds of the anatomical cavity within a 3D volume of discrete voxels representing the Euclidian space system. The length of each side of each voxel is the same and equal to a voxel size.

The determined positions in the position space may be used to define the bounds of an anatomical cavity within a 3D position space, thereby defining an anatomical model. Put another way, the anatomical model may be represented by a 3D volume of discrete voxels, e.g. in the form of a 3D matrix, which define the bounds of the anatomical cavity.

The process of reconstructing an anatomical model from a point cloud is conceptually illustrated in Figure 4, which demonstrates a process 450 in which a cloud of points 410 ("point cloud") is transformed into an anatomical model 420. In the illustrated example, this is performed by creating a (3D) surface from the point cloud data, methods of which will be readily apparent to the skilled person.

For example, a point cloud can be converted into a polygon mesh or triangular mesh model (or other surface model) using a surface reconstruction approach. A variety of suitable approaches is discussed in Berger, Matthew, et al. "A survey of surface reconstruction from point clouds." Computer Graphics Forum. Vol. 26. No. 1. 2017, and further mechanisms will be readily apparent to the skilled person.

Other forms of anatomical model will be apparent to the skilled person.

Historically, the anatomical model is generated from positions in the position space, and is therefore generated in the position/physical space.

The skilled person will appreciate that the derived mapping/transfer function ("V2R function") can also be used to track a location of the internal electrodes with respect to a constructed anatomical model. This facilitates the generation and display of an anatomical model and an indicator of a current location/position of an interventional device 335 mounting internal electrodes 331, 332, 333 with respect to the anatomical model.

Of course, a visual representation of any generated anatomical model and/or determined position may be generated and provided at a user interface 399. Some embodiments provide an anatomical model system or processing arrangement formed of an anatomical model generator and a user interface, at which the anatomical model is generated.

The present disclosure relates to a new process for generating the anatomical model in the position/physical space, and in particular, to a new mechanism for processing the obtained electrical responses to generate the anatomical model.

The present disclosure proposes a concept of generating a response space anatomical model, before mapping the response space anatomical model to the position space. This avoids a requirement to performing mapping of all responses to appropriate positions (in the position space) before an anatomical model is generated. This reduces the amount of mapping that needs to be performed, significantly decreasing a computational complexity of constructing a position space anatomical model.

There is therefore proposed a computer-implemented method for generating an anatomical model generator, as well as a corresponding anatomical model generator. The anatomical model generator may form part of an overall processing system configured to

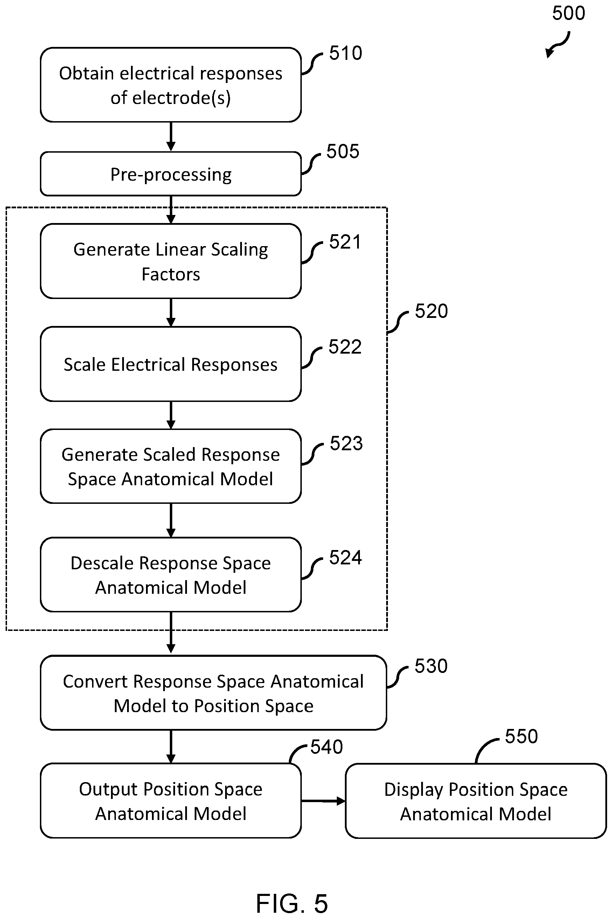

Figure 5 illustrates a method 500 according to an embodiment. The method may be performed by the anatomical model generator 390B, described with reference to Figure 3.

The method 500 comprises a step 510 of receiving or obtaining electrical responses, defining a response space, of one or more electrodes of the interventional device to crossing electric fields induced within the cavity. The electrical responses may be obtained directly from the interventional device itself, or from a memory storing the electrical responses. The electrical responses may be received at an input interface of an anatomical generator.

The method then comprises a process 520 of generating a response space anatomical model using the obtained electrical responses. This process may comprise applying previously described model reconstruction approaches to the electrical responses (or modified electrical responses) in the response space.

In particular, it will be apparent that the electrical responses can each represent a different point within a response space (e.g. values of a response may represent a particular co-ordinate for the response within the response space). A model reconstruction approach, such as a surface reconstruction approach, can be applied to the electrical responses within the response space.

Thus, rather than constructing an anatomical model using positions in a position space, an anatomical model is constructed using (original or modified, as described below) responses in a response space, to thereby produce a response space anatomical model. The process for generating an anatomical model from responses is functionally similar to the process for generating an anatomical model from mapped positions (e.g. as both represent different points in a Euclidian/Cartesian space).

The response space anatomical model is then converted, in a step 530, into a position space anatomical model. This is performed using a first mapping function, e.g. the mapping function generated by the mapping function generator previously described.

Thus, the first mapping function may have been obtained by relating electrical responses of pairs of electrodes to positions in the position space using one or more inter-electrode distances between the pairs of electrodes as constraints. Preferably, the first mapping function is non-linear to account for the non-linearity of the electric fields within the anatomical cavity (caused by differences in the dielectric constant of different tissues or absence of tissue).

The precise operation of step 530 may depend upon the format of the response/position space anatomical model. For instance, where the response space anatomical model comprises a mesh having defined vertices (e.g. representing vertices of triangles forming the mesh), the first mapping function may be applied to each vertex in order to map said vertex into the position space.

Steps 520 and 530 may be carried out by a data processor of an anatomical model generator (which also comprises the input interface for obtaining the electrical responses).

The method 500 may further comprise a step 540 providing a representation of at least part of the position space anatomical model to a user interface. Step 540 may comprise, for example, outputting (a representation of) a position space anatomical model to a user interface via an output interface of an anatomical model generator.

In some examples, the method 500 also comprises displaying, using the user interface, the position space anatomical model. The anatomical model generator and the user interface may together form an anatomical model system or processing arrangement according to an embodiment.

The disclosed method facilitates generation of a position space model without needing to perform accurate mapping of each response (in the response space) to a corresponding position in the position space. Rather, an anatomical model (which is generally formed of fewer data points than the cloud of responses) is mapped into the position space. The number of calculations required to generate the position space anatomical model is therefore reduced, leading to quicker constructions of the position space anatomical model.

Method 500 also illustrates an embodiment for process 520, which provides a position space anatomical model of a higher accuracy.

The process 520 may comprise a step 521 of determining linear scaling factors that (linearly) scale each electrical response to an approximate position, for the electrode associated with that electrical response, in the multidimensional position space. A linear scaling factor is a constant or coefficient that is applied to a value of the electrical response to produce a value for a particular position in the position space.

The number of linear scaling factors may be equal to the number of values/measurements that form a single response. In particular, each value/measurement may have a specific linear scaling factor.

The linear scaling factors could be determined in a number of ways. In one example, the linear scaling factors are determined using the approach suggested in the United States Patent Application having publication number US 5,697,377 A, in which the "constants" or "sensitivities" of this document are used as the linear scaling factors of this application. This approach effectively determines a mapping function that linearly maps a response in response space to a position in position space (i.e. a "linear mapping function"). The constants of this linear mapping function can act as linear scaling factors for the present invention.

Another approach could be to apply determine positions of the plurality of electrodes within the multi-dimensional position space from (e.g. a subset of) the electrical responses using a second mapping function (e.g. similar or identical to the first mapping function); and determining linear scaling factors between the electrical responses, in the response space, and the corresponding positions in the multi-dimensional position space. This latter step may comprise, for instance, dividing positions in the multi-dimensional position space by the linear scaling factors.

By way of example, where an electrical response is a voltage response formed of three voltages, each representing a voltage measurement at an electrode to a specific electric field (e.g. a voltage at a frequency unique to a particular electric field), then an electrical response may be represented by $V = v_x, v_y, v_z$. Put another way, each voltage response $V$ may comprise three measurements identifying a position within the response space, e.g. an x-axis measurement $v_x$ (for a first electric field), a y-axis measurement $v_y$ (for a second electric field) and a z-axis measurement $v_z$ (for a third electric field). Linear scaling factors may be determined (in step 521) for each of the measurements of an electric response to map a measurement at each axis of the response space to a position along a respective axis of the position space. Thus, a response $V$ may be mapped to a position $R$ (having values $r_x, r_y, r_z$) in a position space (i.e. R-space) using linear scaling factors $s_x$, $s_y$ and $s_z$ which are respectively applied to the values of the electrical response.

In this example, step 521 may therefore comprise estimating values $s_x$, $s_y$ and $s_z$ (linear scaling values) such that the equations:

$$r_x = s_x . v_x \qquad (1)$$

$$r_y = s_y . v_x \qquad (2)$$

$$r_z = s_z . v_z \qquad (3)$$

defines a linear function $f(V) = R$ to be applied to all values of $V$ that agree with known inter-electrode distances in the best possible way - i.e. to minimize an error in known inter-electrode distances.

A first approach for calculating such values is disclosed by the United States Patent Application having publication number US 5,697,377 A. The constants or sensitivities of this document may correspond to the linear scaling values $s_x$, $s_y$ and $s_z$ of this document.

A second approach may make use of more complex (e.g. non-linear or machine-learning) mapping functions (such as the first mapping function), i.e. a second mapping function, to generate more accurate identification of the corresponding positions in position space for all responses (or a subset of the known responses for improved processing efficiency) in the response space. The second mapping function may here be equivalent to the first mapping function. Suitable linear scaling values can then be calculated by dividing the values of each positions by the values of the corresponding response, and averaging the quotients.

Thus, for a plurality of voltage responses $V = v_x, v_y, v_z$, which are mapped, using the second mapping function, to a corresponding plurality of positions $R = r_x, r_y, r_z$ the linear scaling factors could be calculated by:

$$s_x = \frac{\sum_{i=1}^{N} \frac{r_{xi}}{v_{xi}}}{N} \tag{4}$$

$$s_y = \frac{\sum_{i=1}^{N} \frac{r_{yi}}{v_{yi}}}{N} \tag{5}$$

$$s_z = \frac{\sum_{i=1}^{N} \frac{r_{zi}}{v_{zi}}}{N} \tag{6}$$

where N is equal to the number of voltage responses mapped using the second mapping function, $v^*_i$ represents a particular voltage response and $r^*_i$ its corresponding positon in position space. * may be replaced by x, y, z where appropriate.

As mentioned above, for this second approach, rather than mapping all responses to the position space (as would be required for a conventional approach to generating a position space anatomical model), a subset (i.e. not all) of the obtained responses may be mapped and used to generate the linear scaling values. This facilitates generation of suitable linear scaling factors without requiring the use of a full first mapping function.

Other approaches for generating linear scaling factors will be apparent to the skilled person. The disclosed approaches may be appropriately modified for different types of responses (e.g. formed of a larger/smaller number of values/measurements and/or different types of values/measurements). The proposed equations and number of linear scaling factors can be modified accordingly.

For instance, in the above examples, the term V may be replaced (where appropriate) with other electrical measurements, such as impedance measurements, capacitance measurements or the like. Any suitable electrical measurement may form part of the electrical response, and an electrical response may include a combination of different measurements.

The process 520 then moves to a step 522 of determining scaled electrical responses from the electrical responses using the linear scaling factors. This may comprise, for instance, applying the linear scaling factors calculated in step 521 to each of the electrical responses to generate the scaled electrical responses.

It should be clear that this step 522 is not equivalent to a mapping of the electrical responses (in the response space) into position (in the position space), as the calculated results do not represent a true position within a position space (due at least to the non-linearity of electric fields induced within a patient).

Thus, each electrical response is scaled. This may, for instance, comprise applying the linear scaling values $s_x$, $s_y$, $s_z$ to the respective values of the response (e.g. $v_x$, $v_y$, $v_z$ respectively). Effectively, this may comprise executing equations 1, 2 and 3 for each of the electrical responses.

Process 520 may then perform a step 523 of generating a scaled response space anatomical model in the electrical response space from the scaled electrical responses. This may comprise, for example, constructing a surface mesh that fits the scaled electrical responses in the response space.

Process 520 may then perform a step 524 of generating the response space anatomical model using the linear scaling factors. In other words, step 524 may comprise descaling the scaled response space anatomical model using the linear scaling factors.

Step 524 can be performed, for example, by identifying a position of each vertex of the anatomical model in the response space, and dividing the position of each vertex by the linear scaling values. It will be clear that each vertex of the anatomical model will have the same number of values as a response (as it lies in a same response space), so that it is possible to descale each vertex using the same linear scaling factors.

By way of example, the anatomical model may be defined using a plurality of shapes (e.g. triangles, squares or shapes with a larger number of sides) that form a surface mesh, each shape being defined by three or more vertices (each vertex representing a position in the response space). Each vertex may be divided by the linear scaling values to generate a descaled vertex.

This produces a (descaled) response space anatomical model.

As before, the produced response space anatomical model can then be processed in a step 530 to generate the position space anatomical model.

Scaling of the electrical responses in this way (before (re)constructing the anatomical model) facilitates use (in the response space) of a reconstruction algorithm designed for reconstructing an anatomical model in the position/physical space. Thus, a same reconstruction algorithm (previously used for generating the anatomical model directly in the position space) can be used. In particular, the scaling provides the responses with position space detail that is desirable for improving or tuning a reconstructed anatomical model to the "real world".

The above-described embodiment of process 520 is not essential. Rather, in some examples, the response space anatomical model could be generated directly using the responses obtained in step 510 (i.e. without scaling or the like). This may make use of a new or adapted reconstruction algorithm for the response space. This approach improves a processing efficiency, but with potential loss of accuracy (as there is the potential to lose some R space characteristics and/or details), as position space reconstruction algorithms have been widely developed and tuned/optimized.

Method 500 may be iteratively repeated, e.g. as new electrical responses become available (e.g. during the course of an imaging process).

In some examples, if performed, step 521 is only performed in the first iteration, or in a limited number of iterations (e.g. once every X iterations, where X is a predetermined number, e.g. no less than 5 or no less than 10), with other iterations using the previously determined linear scaling factors. This reduces the need for performing resourceintensive calculation of linear scaling factors.

In such examples, step 521 may comprise using the second approach, previously described, in which the mapping function is the first mapping function (e.g. one conventionally used to accurately map all responses to the position space before generating the anatomical model). This approach means that fewer iterations of performing the first mapping function on all responses need to be performed, compared to approaches adopted in the prior art, whilst still facilitating the generation of an accurate and regularly updated position space anatomical model.

In some examples, the method 500 further comprises a preprocessing step 590, performed after the electrical responses are obtained. The preprocessing step may comprise applying calibration correction(s) to the obtained electrical responses (e.g. based on a reference response obtained at the reference electrode), performing outlier detection and removal on the obtained electrical responses, performing drift correction on the obtained electrical responses and/or performing gating on the obtained electrical responses.

A gating process may comprise, for instance, only obtaining (or filtering to obtain) electrical responses within a predetermined part of an anatomical cycle of the subject, e.g. during a particular part of a heartbeat or respiratory cycle.

In this way, the electrical responses used in the remainder of method 500 may comprise of only electrical responses (originally sampled) within a predetermined part of an anatomical cycle of the subject.

In some examples, the method 500 is configured to further comprise a step of controlling 506 generation of electrical signals and application of these signals to a set of external body electrodes which when applied to the surface of the subject cause the crossing electric fields induced within the cavity. In other words, the method 500 may also comprise/perform the functions of the electric field generator 330.

The method 500 may also comprise controlling 507 the obtaining of the electrical responses of the one or more electrodes. Thus, the method 500 may comprise controlling the sampling of the electrical responses from the one or more electrodes.

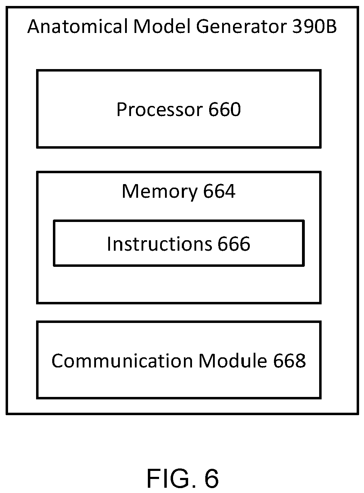

Figure 6 is a schematic diagram of an anatomical model generator 390B, according to embodiments of the present disclosure. The anatomical model generator 390B may be adapted to carry out any method herein described, e.g. as previously set out.

As shown, the anatomical model generator 390B may include a (data) processor 660, a memory 664, and a communication module 668. These elements may be in direct or indirect communication with each other, for example via one or more buses.

The processor 660 may include a central processing unit (CPU), a digital signal processor (DSP), an ASIC, a controller, an FPGA, another hardware device, a firmware device, or any combination thereof configured to perform the operations described herein. The processor 660 may also be implemented as a combination of computing devices, e.g., a combination of a DSP and a microprocessor, a plurality of microprocessors, one or more microprocessors in conjunction with a DSP core, or any other such configuration. In some embodiments, the processor is a distributed processing system, e.g. formed of a set of distributed processors.

The memory 664 may include a cache memory (e.g., a cache memory of the processor 660), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and nonvolatile memory, or a combination of different types of memory. In an embodiment, the memory 664 includes a non-transitory computer-readable medium. The non-transitory computer-readable medium may store instructions. For example, the memory 664, or non-transitory computer-readable medium may have program code recorded thereon, the program code including instructions for causing the anatomical model generator 390B, or one or more components of the anatomical model generator 390B, particularly the processor 660, to perform the operations described herein. For example, the anatomical model generator 390B can execute operations of the method 700. Instructions 666 may also be referred to as code or program code.

The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements. The memory 664, with the code recorded thereon, may be referred to as a computer program product.

The communication module 668 can include any electronic circuitry and/or logic circuitry to facilitate direct or indirect communication of data between the processing system 390, the penetration device and/or the user interface (or other further device). In that regard, the communication module 668 can be an input/output (I/O) device. In some instances, the communication module 668 facilitates direct or indirect communication between various elements of the anatomical model generator and/or the system (Figure 3). Thus, the communication module 668 comprises at least an input interface for receiving electrical responses (e.g. directly from the electrodes or from a memory). Similarly, the communication module may comprise an output interface for providing a (representation of) position space anatomical model to a user interface.

The anatomical model generator 390B may form part of a larger anatomical model system or processing arrangement according to an embodiment. The anatomical model system or processing arrangement may comprise a user interface for displaying a representation of the anatomical model (e.g. to a user).

It will be understood that disclosed methods are preferably computer-implemented methods. As such, there is also proposed the concept of a computer program comprising computer program code for implementing any described method when said program is run on a processing system, such as a computer or a set of distributed processors.

Different portions, lines or blocks of code of a computer program according to an embodiment may be executed by a processing system or computer to perform any herein described method. In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

The present disclosure proposes a computer program (product) comprising instructions which, when the program is executed by a computer or processing system, cause the computer or processing system to carry out (the steps of) any herein described method. The computer program (product) may be stored on a non-transitory computer readable medium.

Similarly, there is also proposed a computer-readable (storage) medium comprising instructions which, when executed by a computer or processing system, cause the computer or processing system to carry out (the steps of) any herein described method. There is also proposed computer-readable data carrier having stored thereon the computer program (product) previously described. There is also proposed a data carrier signal carrying the computer program (product) previously described.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. A computer-implemented method (500) for generating a position space anatomical model (420), being an anatomical model in a multidimensional position space, of an anatomical cavity of a subject in which an interventional device (335) comprising one or more electrodes (331, 332, 333) is positioned, the computer implemented method comprising:

   receiving or obtaining (510) electrical responses, defining a response space, of one or more electrodes of the interventional device to crossing electric fields induced within the cavity;
   generating (520) a response space anatomical model using the obtained electrical responses; and
   generating (530) the position space anatomical model by converting the response space anatomical model into the multi-dimensional position space using a first mapping function,
   wherein the computer-implemented method optionally further comprises providing (540, 550) a representation of at least part of the position space anatomical model to a user interface (399).

2. The computer-implemented method of claim 1, wherein the step (520) of generating the response space anatomical model comprises:

   determining (521) linear scaling factors that scale each electrical response to an approximate position, for the electrode associated with that electrical response, in the multidimensional position space;
   determining (522) scaled electrical responses from the electrical responses using the linear scaling factors;
   generating (523) a scaled response space anatomical model in the electrical response space from the scaled electrical responses; and
   generating (524) the response space anatomical model by descaling the scaled response space anatomical model using the linear scaling factors.

3. The computer-implemented method of claim 2, wherein the step of determining linear scaling factors comprises:

   determining positions of the plurality of electrodes within the multidimensional position space from the electrical responses using a second mapping function; and
   determining linear scaling factors between the electrical responses, in the response space, and the corresponding positions in the multi-dimensional position space.

4. The computer-implemented method of claim 3, wherein the step of determining linear scaling factors comprises dividing the positions, in the multi-dimensional position space, by their corresponding electrical responses.

5. The computer-implemented method (500) of claim 2, wherein the step of determining linear scaling factors comprises determining a linear mapping function that maps a response, in response space, to a position in position space using the linear scaling factors.

6. The computer-implemented method of any of claims 1 to 5, wherein the first mapping function is a mapping function obtained by relating electrical responses of pairs of electrodes to positions in the position space using one or more inter-electrode distances between the pairs of electrodes as constraints.

7. The computer-implemented method of any of claims 1 to 6, wherein the response space anatomical model and the position space anatomical model both comprise of a mesh having triangular or quadrilateral cell shapes.

8. The computer-implemented method of any of claims 1 to 7, wherein the electrical responses are electrical responses within a predetermined part of an anatomical cycle of the subject.

9. The computer-implemented method of any of claims 1 to 8, wherein the computer-implemented method comprises:

   controlling generation of electrical signals and application of these signals to a set of external body electrodes which when applied to the surface of the subject cause the crossing electric fields to be induced within the cavity; and/or
   controlling the obtaining of the electrical responses of the one or more electrodes.

10. A computer program product comprising computer program code means which, when executed on a computing device having a processing system, cause the processing system to perform all of the steps of the method (500) according to any of the previous claims.

11. A non-transitory computer readable medium comprising the computer program product of claim 10.

12. An anatomical model generator (390B) for generating an anatomical model (420) in a multidimensional position space of an anatomical cavity of a subject in which an interventional device (335) comprising one or more electrodes (331, 332, 333) is positioned, the anatomical model generator comprising:

   an input interface (668) configured to receive or obtain (510) electrical responses, defining a response space, of one or more electrodes of the interventional device (335) to crossing electric fields induced within the cavity;
   a data processor (660) configured to:

      generate (520) a response space anatomical model using the obtained electrical responses; and
      generate (530) the position space anatomical model by converting the response space anatomical model into the multi-dimensional position space using a first mapping function,

wherein the anatomical model generator optionally further comprises:
an output interface configured to provide (540, 550) a representation of at least part of the position space anatomical model to a user interface.

13. The anatomical model generator of claim 12, wherein the data processor is configured to generate (520) the response space anatomical model by:

determining (521) linear scaling factors that scale each electrical response to an approximate position, for the electrode associated with that electrical response, in the multidimensional position space;
determining (522) scaled electrical responses from the electrical responses using the linear scaling factors;
generating (523) a scaled response space anatomical model in the electrical response space from the scaled electrical responses; and
generating (524) the response space anatomical model by descaling the scaled response space anatomical model using the linear scaling factors.

14. The anatomical model generator of claim 13, wherein the data processor is configured to determine linear scaling factors by:

determining positions of the plurality of electrodes within the multidimensional position space from the electrical responses using a second mapping function; and
determining linear scaling factors between the electrical responses, in the response space, and the corresponding positions in the multi-dimensional position space.

15. An anatomical model system comprising:

the anatomical model generator of any of claims 12 to 14, wherein the anatomical model generator comprises the output interface; and
the user interface (399) configured to receive the representation of at least part of the position space anatomical model and display the received representation.

FIG. 1

FIG. 2

FIG. 3

FIG. 4

500

Obtain electrical responses
of electrode(s) — 510

↓

Pre-processing — 505

Generate Linear Scaling
Factors — 521

↓

Scale Electrical Responses — 522

520

↓

Generate Scaled Response
Space Anatomical Model — 523

↓

Descale Response Space
Anatomical Model — 524

↓

Convert Response Space Anatomical
Model to Position Space — 530

↓

Output Position Space
Anatomical Model — 540 → Display Position Space
Anatomical Model — 550

FIG. 5

Anatomical Model Generator 390B

Processor 660

Memory 664

Instructions 666

Communication Module 668

FIG. 6

<table>
<tr><td>Europäisches Patentamt<br>European Patent Office<br>Office européen des brevets</td><td colspan="2">**EUROPEAN SEARCH REPORT**</td><td>Application Number<br>EP 21 16 8593</td></tr>
</table>

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2020/212527 A1 (NAVIX INT LTD) 22 October 2020 (2020-10-22) * paragraphs [0010], [0065], [0090] - [0096]; figures 4-6 * ----- | 1,10-12 | INV. G16H50/00 G06T19/00 A61B5/367 A61B5/06 |
| Y | WO 2019/034944 A1 (NAVIX INT LTD) 21 February 2019 (2019-02-21) * page 17, line 23 - page 19, line 6 * * page 22, line 9 - page 23, line 31 * * page 46, line 23 - page 52, line 21 * * page 70, line 10 - page 72, line 23 * * figure 13 * ----- | 1,10-12 | A61B5/0538 A61B5/00 |
| A | US 2020/000368 A1 (BEN-HAIM SHLOMO [IT] ET AL) 2 January 2020 (2020-01-02) * paragraphs [0137], [0186], [0187], [0216] - [0222]; figures 5,6 * ----- | 1-15 | |
| A | WO 2017/192775 A1 (ACUTUS MEDICAL INC [US]) 9 November 2017 (2017-11-09) * paragraphs [0200] - [0209] * ----- | 1-15 | |
| A | WO 2020/008416 A1 (NAVIX INT LTD) 9 January 2020 (2020-01-09) * page 44, lines 1-30; figure 15 * ----- | 1-15 | TECHNICAL FIELDS SEARCHED (IPC)<br><br>G16H<br>G06T<br>A61B |

The present search report has been drawn up for all claims

| Place of search | Date of completion of the search | Examiner |
|---|---|---|
| Berlin | 22 September 2021 | Pohjamo, Terhi |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 16 8593

This annex lists the patent family members relating to the patent documents cited in the above-mentioned European search report.
The members are as contained in the European Patent Office EDP file on
The European Patent Office is in no way liable for these particulars which are merely given for the purpose of information.

22-09-2021

| Patent document cited in search report | | Publication date | Patent family member(s) | | | Publication date |
|---|---|---|---|---|---|---|
| WO 2020212527 | A1 | 22-10-2020 | NONE | | | |
| WO 2019034944 | A1 | 21-02-2019 | CN | 111163692 | A | 15-05-2020 |
| | | | EP | 3668393 | A1 | 24-06-2020 |
| | | | WO | 2019034944 | A1 | 21-02-2019 |
| US 2020000368 | A1 | 02-01-2020 | CN | 110461227 | A | 15-11-2019 |
| | | | US | 2020000368 | A1 | 02-01-2020 |
| | | | WO | 2018146613 | A2 | 16-08-2018 |
| WO 2017192775 | A1 | 09-11-2017 | AU | 2017260453 | A1 | 22-11-2018 |
| | | | CA | 3022806 | A1 | 09-11-2017 |
| | | | CN | 109715055 | A | 03-05-2019 |
| | | | EP | 3451917 | A1 | 13-03-2019 |
| | | | JP | 2019514578 | A | 06-06-2019 |
| | | | US | 2019200886 | A1 | 04-07-2019 |
| | | | WO | 2017192775 | A1 | 09-11-2017 |
| WO 2020008416 | A1 | 09-01-2020 | US | 2021259572 | A1 | 26-08-2021 |
| | | | WO | 2020008416 | A1 | 09-01-2020 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5697377 A
- EP 3568068 A
- WO 2018130974 A
- EP 0775466 A2
- EP 3568068 A1
- EP 3607879 A1

**Non-patent literature cited in the description**

- **BERGER, MATTHEW et al.** A survey of surface reconstruction from point clouds. *Computer Graphics Forum,* 2017, vol. 26 (1